# FASCICULE DE BREVET EUROPEEN

(11) **EP 0 577 516 B1**
(45) Date de publication et mention de la délivrance du brevet: **16.10.1996**
(21) Numéro de dépôt: 93401711.2
(22) Date de dépôt: 01.07.1993
(51) Int. Cl.: A61K 7/48, A61K 31/215

(54) **Composition cosmétique et/ou dermatologique à action dépigmentante contenant un acide di- ou tri-caféoylquinique ou un mélange de ceux-ci**
Kosmetische oder dermatologische Zusammensetzung zur Hautdepigmentierung enthaltend eine di- oder tri-Kaffeoylchinasäure oder eine Mischung davon
Cosmetic and/or dermatological composition for skin whitening containing a di- or tri-caffeoylquinic acid or a mixture thereof

(30) Priorité: 01.07.1992 FR 9208091
(43) Date de publication de la demande: 05.01.1994
(73) Titulaire: L'OREAL, F-75008 Paris (FR)
(72) Inventeur: Li, Ming, F-91190 Gif Sur Yvette (FR); Sevenet, Thierry, F-75018 Paris (FR); Schaller, Hubert, F-67600 Selestat (FR); Abdul Hadi, Hamid, F-91190 Gif Sur Yvette (FR); Guenard, Daniel, F-92120 Montrouge (FR); Potier, Pierre, F-75007 Paris (FR); Pilleux, Eric, F-75014 Paris (FR)
(74) Mandataire: Stalla-Bourdillon, Bernard

(56) Documents cités:
- WO-A-91/05543
- PATENT ABSTRACTS OF JAPAN vol. 16, no. 470 (C-990)30 Septembre 1992
- S.T.N. Serveur de bases de donnees, Karlsruhe, DE, Fichier Chemical Abstracts, vol 79, no. 27209
- S.T.N. Serveur de bases de donnees, Karlsruhe, DE, Fichier Chemical Abstracts vol 67, no. 57236

## Description

La présente demande a pour objet une composition cosmétique ou dermatologique à action dépigmentante contenant en tant que principe actif au moins un acide di-ou tri-caféoylquinique. La composition selon l'invention est destinée à blanchir la peau ou à traiter les tâches pigmentaires.

On rappellera que le mécanisme de formation de la pigmentation de la peau, c'est-à-dire de la formation des mélanines est particulièrement complexe et fait intervenir schématiquement les principales étapes suivantes:

Tyrosine → Dopa → Dopaquinone → Dopachrome → Mélanines

l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés, en particulier ses éthers tels que le monomméthyléther d'hydroquinone.

Ces composés, s'ils ont une efficacité certaine, ne sont malheureusement pas exempts d'effets secondaires, ce qui peut rendre leur emploi délicat, voire dangereux.

Ainsi, l'hydroquinone dont l'emploi est d'ailleurs limité à une concentration de 2%, est un composé particulièrement irritant et cytotoxique pour le mélanocyte dont le remplacement, total ou partiel, a été envisagé par de nombreux auteurs.

Il a été ainsi proposé l'emploi de diverses autres substances notamment l'acide caféïque et/ou ses esters ou amides. Ces compositions à base d'acide caféïque ou dérivés font l'objet de la demande de brevet français n° 89.13774 (2.653.336).

Des substances naturelles ont aussi été proposées dont l'arbutoside et le méthylarbutoside. Ces compositions font l'objet du brevet français n° 85.04288 (2.577.805).

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines, soit en inhibant un des enzymes impliqués, soit en s'intercalant comme analogue structural dans la voie de synthèse qui peut ainsi être bloquée, d'où l'effet dépigmentant.

L'utilisation de substances dépigmentantes par voie topique présentant une bonne efficacité et étant inoffensive est tout particulièrement recherchée en vue de traiter les hyperpigmentations régionales par hyperactivité mélanocytaire telles que les mélasmas idiopathiques, survenant lors de la grossesse (masque de grossesse ou chloasma) ou secondaires à la contraception oestro-progestative, les hyperpigmentations localisées par hyperactivité et prolifération mélanocytaire bénigne telles que les taches pigmentaires séniles dites lintigo actiniques, les hyperpigmentations accidentelles telles que la photosensibilisation et la cicatrisation post-lésionnelle, ainsi que certaines leucodermies telles que le vitiligo ou, à défaut de pouvoir repigmenter la peau lésée, on achève de dépigmenter les zones de peau normales résiduelles pour donner à l'ensemble de la peau une teinte blanche homogène.

Après de nombreux travaux sur diverses substances naturelles, on a constaté que certains acides caféoylquiniques avaient une action dépigmentante particulièrement prononcée et bien supérieure à celle de l'acide caféïque ou ses alkyl esters et à celle d'autres substances dépigmentantes connues telles que l'hydroquinone.

Ces propriétés dépigmentantes ont pu être mises en évidence par le test d'inhibition "in vitro" de l'activité de la tyrosinase.

La présente invention a donc pour objet une utilisation en tant que composé actif, à usage topique, d'au moins un acide di- ou tri-caféoylquinique correspondant à la formule générale (I) suivante : 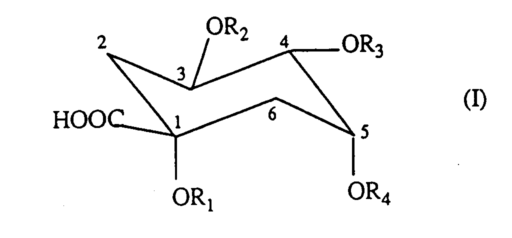 dans laquelle :
R₁, R₂, R₃ et R₄ représentent un atome d'hydrogène ou le radical CAF de formule : 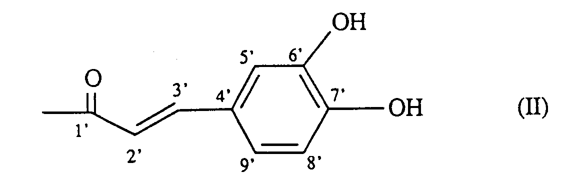 sous réserve que deux au moins et trois au plus des radicaux R₁, R₂, R₃ ou R₄ représentent le radical CAF mais à l'exclusion de R₁ et R₄ représentant le radical CAF et R₂ et R₃ représentant un atome d'hydrogène,
ou un mélange desdits acides caféoylquiniques,
pour la préparation d'une composition dermatologique destinée au traitement spécifique des taches pigmentaires et à blanchir la peau.

Parmi les composés selon l'invention de formule (I), on peut citer :
- l'acide 4,5-di-caféoylquinique,
- l'acide 3,5-di-caféoylquinique,
- l'acide 1,3-di-caféoylquinique,
- l'acide 3,4-di-caféoylquinique,
- l'acide 3,4,5-tri-caféoylquinique, et
leurs mélanges.

La plupart des composés couverts par la formule générale (I), des compositions selon l'invention ont été décrits dans la littérature, notamment en tant que constituants de *Chrysothamnus Paniculatus* par Timmermann et al dans J.NAT.PROD., 83, Vol.46(3), pp.365-368.

Les propriétés bénéfiques de certains de ces composés les ont fait utiliser en médecine populaire (extraits des espèces argentines d'ASTERAGEAE, réf: Acta Farm. Bonaerensa, 89, Vol.8(1), pp.3-9).

Ils ont également été décrits en tant qu'agents antiallergiques dans la demande de brevet japonais JP 88-218619 et en tant qu'agents anti-grippe dans la demande de brevet japonais JP 85-243016, lorsqu'ils sont obtenus par extraction méthanolique de graines de *Helianthus Annus.*

Les compositions selon l'invention contiennent en général une concentration en principe actif de formule (I) comprise entre 0,01 et 15 % en poids et de préférence entre 0,1 et 8 % en poids par rapport au poids total de la composition.

Les compositions selon l'invention peuvent se présenter sous diverses formes, notamment de solutions aqueuses ou hydroalcooliques, d'émulsions du type huile-dans-l'eau (H/E) ou eau-dans-l'huile (E/H) ou encore sous forme de gels émulsionnés.

De préférence, les compositions selon l'invention se présentent sous forme d'une lotion, d'une crème, d'un lait, d'un gel, d'un masque, ou de dispersions vésiculaires, ces vésicules pouvant être constituées de lipides ioniques (liposomes) et/ou de lipides non-ioniques.

Dans les émulsions, la phase grasse peut être constituée d'une huile végétale ou animale, d'une huile minérale ou encore d'une huile synthétique.

Parmi les huiles végétales ou animales modifiées ou non, on peut citer notamment l'huile d'amande douce, l'huile d'avocat, l'huile de ricin, l'huile d'olive, l'huile de jojoba, le perhydrosqualène, l'huile de Calophyllum, la lanoline et ses dérivés, l'huile de tournesol, l'huile de germe de blé, l'huile de sésame, l'huile d'arachide, l'huile de pépins de résin, l'huile de soja, l'huile de colza, l'huile de carthame, l'huile de coco, l'huile de maïs, l'huile de noisette, le beurre de karité, la graisse de Shorea robusta, l'huile de palme et l'huile de noyau d'abricot.

Parmi les huiles minérales, on peut citer par exemple l'huile de vaseline et parmi les huiles synthétiques, les palmitates d'éthyle et d'isopropyle, les myristates d'alkyle tels que le myristate d'isopropyle, de butyle, de cétyle, le stéarate d'hexyle, les triglycérides des acides octanoïque et décanoïque (par exemple le produit vendu sous la dénomination de "Miglyol®" par la Société Dynamit Nobel), le ricinoléate de cétyle, l'octanoate de stéaryle (huile de purcellin) et le polyisobutène hydrogéné ainsi que des cires telles que l'ozokérite.

L'excipient gras peut également contenir certains composés considérés comme des produits gras à savoir des alcools à longues chaînes tels que l'alcool cétylique, l'alcool stéarylique, l'alcool myristique, l'alcool hydrostéarylique, l'alcool oléïque ou l'alcool isostéarylique.

Comme huile synthétique, on peut notamment citer les huiles de silicone. Parmi celles-ci, on utilise de préférence le cyclopentadiméthylsiloxane et notamment le produit vendu sous la dénomination de "Volatil Silicone 7158®" par la Société Union Carbide, ainsi que l'alkyldiméthicone copolyol, en particulier le produit vendu sous la dénomination de "Abil WE 09®" par la Société Goldschmidt.

Les compositions cosmétiques ou dermatologiques selon l'invention peuvent bien entendu contenir d'autres ingrédients conventionnels tels que des agents humectants, des conservateurs, des colorants, des parfums, des agents de pénétration tels que le monoéthyléther de diéthylèneglycol.

Ces compositions sont appliquées par voie topique en quantité correspondant aux doses d'application usuelles pour le type de composition considérée (gels, crèmes, lotions.....). Par exemple, dans le cas d'une crème, on utilise de 0,5 à 3 mg et notamment de 1 à 2 mg de crème par cm² de peau et par application à raison d'une ou deux applications par jour.

### Préparation des composés selon l'invention par extraction à partir d'écorce sèche de Scaevola koenigii, Goodeniaceae, plante de Malaisie

100 g d'écorce sèche de *Scaevola koenigii,* Goodeniaceae récoltée en Malaisie sont extraits par du méthanol (température à 50-60°C) à quatre reprises. Les filtrats méthanoliques réunis sont évaporés à sec. A partir de 100 g d'écorce sèche, on obtient 18,5 g d'un extrait (soit un rendement de 18,5 %) qui est soumis à différents traitements comme indiqué ci-après dans le Tableau I pour obtenir un mélange d'acides di- et tri-caféoylquiniques.

Des dérivés d'acide caféoylquinique, actifs sur la tyrosinase, sont séparés avec les autres composés en réglant le pH. En raison de l'existence d'une fonction acide carboxylique dans ces composés, leur solubilité dans l'eau ou le butanol dépend du pH du milieu. Quand le pH est supérieur à 7, l'acide est sous forme de sel (RCOO⁻) qui se dissout dans l'eau. Par contre, il est soluble dans le butanol quand le pH est inférieur à 6. Pour cette raison, il peut être transféré de l'eau vers le butanol en réglant le pH du milieu.

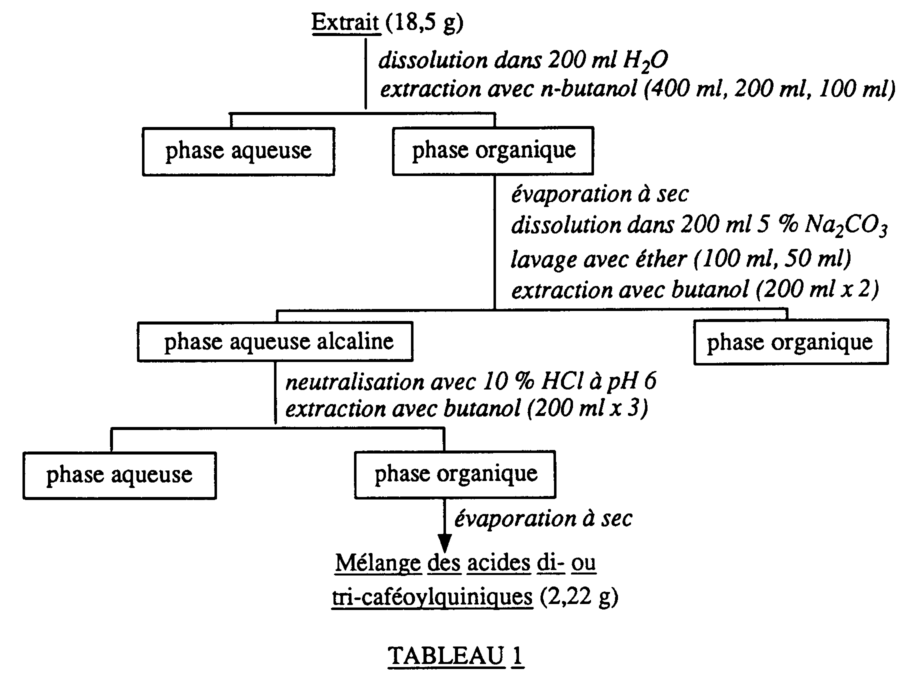

18,5 g de l'extrait brut sont dissous dans 200 ml d'eau. On extrait 3 fois avec du n-butanol (400, 200, 100 ml). Les phases butanoliques réunies sont évaporées à sec et reprises dans 200 ml d'une solution de Na₂CO₃ à 5 %. La phase alcaline est lavée avec de l'éther (100, 50 ml) puis extraite avec 2 fois 200 ml de butanol. Puis la phase alcaline est neutralisée jusqu'à pH 6 avec 10 % HCl, réextraite avec le butanol. Les phases butanoliques réunies sont évaporées à sec. On obtient 2,22 g d'un mélange d'acides di- et tri-caféoylquiniques sous forme d'une poudre jaune.

A partir du mélange, on isole par CLHP préparative différentes fractions dont les composés ont été identifiés quant à leur structure par ¹H RMN dans le méthanol deutérié et par spectre de masse.

Ces composés ainsi isolés sont essentiellement ceux précédemment énumérés et sont obtenus avec des rendements variables en fonction de leur structure.

Les conditions de la CLHP préparative ont été les suivantes : Prep Pak -45 x 300mm, deltaPak C₁₈15 µm. Débit 80 ml/min. Solvant d'élution A : 25 % MeOH, 1 % d'acide acétique. Solvant d'élution B : 40 % MeOH, 1 % d'acide acétique. Gradient linéraire du solvant A à B pendant 30 minutes. Ensuite élution avec 100 % de solvant B pendant 50 minutes.

### Etude "in vitro"

Les acides di- et tri-caféoylquiniques de formule générale (I) ont été étudiés comparativement à l'acide caféïque à quantité molaire équivalente dans le test d'inhibition in vitro de l'activité de la tyrosinase.

Selon ce test, on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxyation non enzymatiques aboutissant à la formation des mélanines.

On mesure donc la concentration en dopachrome formée au cours du temps en présence et en absence de l'inhibiteur.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

Pour chaque composé, on mesure sa teneur en micromoles par litre conduisant à 50% d'inhibition de la tyrosinase (IC 50).

### Protocole expérimental :

### Réactifs:

A - Tampon, phosphate 0,1 M pH = 6,5 (composition pour 200 ml de tampon: 68,5ml de NaH₂PO₄ 0,2 M + 31,5 ml de Na₂HPO₄ 0,2 M + 100 ml d'eau)
B - Solution mère de L-tyrosine à 2.10⁻³M dans A
C - Solution mère de L-dopa à 10⁻⁴M dans A
D - Solution mère de tyrosinase de champignons à 2.400 unités/ml dans A
E - Solution mère de l'inhibiteur à 10⁻²M dans l'éthanol (les solutions C et D sont à préparer le jour même).

### Résultats :

| | |
|---|---|
| - cuve de référence : | 3 ml de A |
| - cuve d'essai : | 1,75 ml de A + 1 ml de B |
| | 0,1 ml de C |
| | 0,1 ml de E |
| - homogénéiser et équilibrer à 25°C, | |
| - ajouter 0,05 ml de D dans la cuve d'essai, | |
| - mélanger rapidement et observer la cinétique par la mesure de l'absorbance 475 nm en fonction du temps. | |

**TABLEAU II**

| ***Composés*** | ***IC 50*** |
|---|---|
| Acide caféïque (réference) | 530 |
| Acide 4,5-di-caféoylquinique | 45 |
| Acide 3,5-di-caféoylquinique | 52 |
| Acide 1,3-di-caféoylquinique | 47 |
| Acide 3,4-di-caféoylquinique | 85 |
| Acide 3,4,5-tri-caféoylquinique | 29 |

Comme on peut le constater les acides di- et tri-caféoylquiniques des compositions selon l'invention ont une activité inhibitrice de la mélanogénèse nettement supérieure à celle de l'acide caféïque.

On va maintenant donner à titre d'illustration plusieurs exemples de compositions cosmétiques et dermatologiques selon l'invention.

### COMPOSITIONS COSMETIOUES OU DERMATOLOGIQUES

### EXEMPLE 1: Lotion

| | |
|---|---|
| - Alcool | 50% |
| - Polyoxyéthylèneglycol (PEG 8) | 30% |
| - Ethoxy-diglycol | 5% |
| - Glycérine | 5% |
| - Eau | 2% |
| - Acide 1,3-di-caféoyquinique | 8% |

### EXEMPLE 2: Emulsion eau-dans-l'huile

| | |
|---|---|
| - Propylèneglycol | 10% |
| - Acide 3,4,5-tri-caféoylquinique | 4% |
| - Huile de vaseline | 20% |
| - Alkyldiméthicone copolyol sous le dénomination de "Abil WE 09®" par la Société Goldschmidt | 3% |
| - Conversateur | 0,2% |
| - Parfum | 0,1% |
| - Eau | qsp 100% |

### EXEMPLE 3: Emulsion huile-dans-l'eau

| | |
|---|---|
| - Propylèneglycol | 10% |
| - Acide 4,5-di-caféoylquinique | 4% |
| - Huile de vaseline | 20,5% |
| - Sorbitan isostéarate | 5% |
| - Stéaryldiméthylbenzylammonium hectorite vendu sous la dénomination de "Miglyol Gel®" par la Société Huls | 5% |
| - Triglycérides des acides caprique et caprylique | 1% |
| - Conversateur | 0,2% |
| - Parfum | 0,1% |
| - Eau | qsp 100% |

### EXEMPLE 4 : Préparation de liposomes en formulation sérum

Dans un ballon rond de 1 litre, on pèse les produits suivants :

| | |
|---|---|
| - Lécithine de soja vendue sous la dénomination de "Lipoïd S75®" par la Société Lipoïds | 3,0% |
| - Acide 3,4-di-caféoyolquinique | 0,3% |

que l'on dissout dans 100 ml d'un mélange de solvants chloroforme-méthanol dans le rapport 2/1.

On évapore le solvant à l'aide d'un évaporateur rotatif et on élimine les dernières traces de solvant par passage à la pompe à palettes pendant 1 heure. On met en contact l'assocation de lipides obtenue avec 60 g d'eau déminéralisée contenant 1 g de glycérine, et on homogénéise le mélange à 40°C, à l'aide d'un homogénéisateur Virtis.

On termine la formule en ajoutant les produits suivants :

| | |
|---|---|
| - Parahydroxybenzoate de méthyle | 0,2 g |
| - Polymère d'acide carboxyvinylique vendu sous la dénomination de "Carbopol 940®" par la Société Goodrich | 0,1 g |
| - Triéthanolamine | qs pH = 6 |
| - Eau | qsp 100 g |

On obtient ainsi un sérum opalescent fluide mais ne s'écoulant pas.

### EXEMPLE 5 : Préparation de vésicules non ioniques en formulation crème

Dans un ballon rond de 100 ml, on pèse les produits suivants :
- Lipide non-ionique de formule :

   C₁₂H₂₅-[OC₂H₃(R)]-O-[C₃H₅(OH)O]ₙ-H

   où -OC₂H₃(R)- est constitué par un mélange des radicaux : 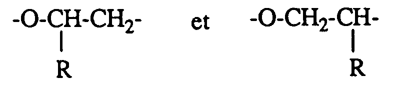 où -C₃H₅(OH)-O- est constitué par un mélange des radicaux : 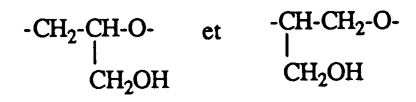 où n = 6 ;

| | |
|---|---|
| et où R est un mélange des radicaux C₁₄H₂₉ et C₁₆H₃₃ | 0.9 g |
| - Acylglutamate de sodium HS21® (vendu par la Société Ajinomoto) | 0,1 g |
| - Acide 3,4,5-tri-caféoylquinique | 0,1 g |

que l'on dissout dans 30 ml d'un mélange de solvants chloroforme-méthanol dans le rapport 2/1.

On évapore le solvant à l'aide d'un évaporateur rotatif et on élimine les dernières traces de solvant par passage à la pompe à palettes pendant 1 heure.

On met au contact l'association de lipides obtenue avec 40 g d'eau déminéralisée contenant 3 g de glycérine, et on homogénéise le mélange à 40°C, à l'aide d'un homogénéisateur Virtis.

On ajoute ensuite 10 g de perhydrosqualène et on homogénéise le mélange à la température ambiante à l'aide du Virtis.

On finit la formule en ajoutant les produits suivants :

| | |
|---|---|
| - Parahydroxybenzoate de méthyle | 0,1 g |
| - Polymère d'acide carboxyvinylique vendu sous la dénomination de "Carbopol 940®" par le Société Goodrich | 0,4 g |
| - Triéthanolamine | qs pH = 6 |
| - Eau | qsp 100 g |

On obtient ainsi une crème blanche, épaisse.

## Revendications

1. Utilisation en tant que composé actif, à usage topique, d'au moins un acide di- ou tri-caféoylquinique correspondant à la formule générale (I) suivante : 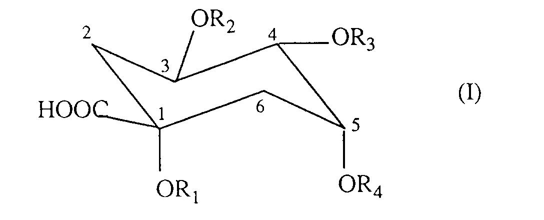 dans laquelle :
R₁, R₂, R₃ et R₄ représentent un atome d'hydrogène ou le radical CAF de formule : 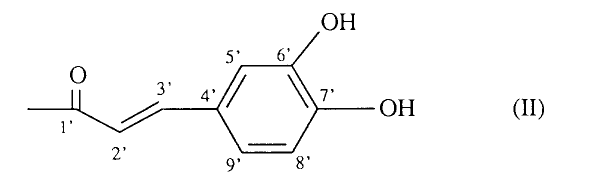 sous réserve que deux au moins et trois au plus des radicaux R₁, R₂, R₃ ou R₄ représentent le radical CAF mais à l'exclusion de R₁ et R₄ représentant le radical CAF et R₂ et R₃ représentant un atome d'hydrogène,
ou un mélange desdits acides caféoylquiniques,
pour la préparation d'une composition dermatologique destinée au traitement spécifique des taches pigmentaires et à blanchir la peau.

2. Utilisation selon la revendication 1, caractérisée par le fait que la composition est utilisée par voie topique à raison de 0,5 à 3 mg par cm² de peau.

3. Utilisation selon l'une quelconque des revendications 1 et 2, caractérisée par le fait que l'acide di- ou tri-caféoylquinique est choisi parmi :
- l'acide 4,5-di-caféoylquinique,
- l'acide 3,5-di-caféoylquinique,
- l'acide 1,3-di-caféoylquinique,
- l'acide 3,4-di-caféoylquinique,
- l'acide 3,4,5-tri-caféoylquinique, et
leurs mélanges.

4. Utilisation selon l'une quelconque des revendications précédentes, caractérisée par le fait que l'acide di- ou tri-caféoylquinique est présent en une concentration comprise entre 0,01 et 15 % en poids par rapport au poids total de la composition.

5. Utilisation selon l'une quelconque des revendications précédentes, caractérisée par le fait que l'acide di- ou tri-caféoylquinique est présent dans la composition en une concentration comprise entre 0,1 et 8 % en poids par rapport au poids total de la composition.

6. Utilisation selon l'une quelconque des revendications précédentes, caractérisée par le fait que la composition se présente sous forme d'une lotion, d'une crème, d'un lait, d'un gel, d'un masque, ou de dispersions vésiculaires constituées de lipides ioniques (liposomes) et/ou de lipides non-ioniques.

7. Utilisation selon l'une quelconque des revendications précédentes, caractérisée par le fait que la composition contient en outre au moins un ingrédient dermatologique choisi parmi les agents humectants, les agents conservateurs, les colorants, les parfums et les agents de pénétration.

## Claims

1. Use, as active compound for topical use, of at least one di- or tricafeoylquinic acid corresponding to the following general formula 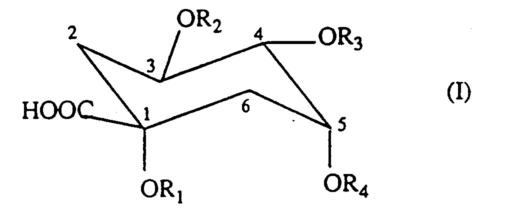 in which:
R₁, R₂, R₃ and R₄ represent a hydrogen atom or the CAF radical of formula: 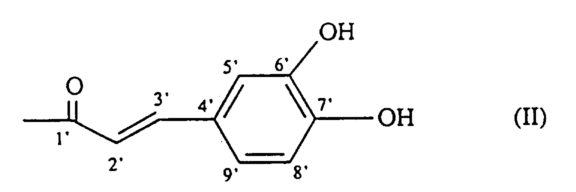 provided that at least two and at most three of the radicals R₁, R₂, R₃ or R₄ represent the CAF radical, but with the exclusion of R₁ and R₄ representing the CAF radical and R₂ and R₃ representing a hydrogen atom,
or a mixture of the said cafeoylquinic acids,
for the preparation of a dermatological composition intended for the specific treatment of pigmented spots and for whitening the skin.

2. Use according to Claim 1, characterized in that the composition is used topically in an amount of 0.5 to 3 mg per cm² of skin.

3. Use according to either of Claims 1 and 2, characterized in that the di- or tricafeoylquinic acid is chosen from:
- 4,5-dicafeoylquinic acid,
- 3,5-dicafeoylquinic acid,
- 1,3-dicafeoylquinic acid,
- 3,4-dicafeoylquinic acid,
- 3,4,5-tricafeoylquinic acid,
and mixtures thereof.

4. Use according to any one of the preceding claims, characterized in that the di- or tricafeoylquinic acid is present at a concentration of between 0.01 and 15 % by weight relative to the total weight of the composition.

5. Use according to any one of the preceding claims, characterized in that the di- or tricafeoylquinic acid is present in the composition at a concentration of between 0.1 and 8 % by weight relative to the total weight of the composition.

6. Use according to any one of the preceding claims, characterized in that the composition is provided in the form of a lotion, a cream, a milk, a gel, a pack, or vesicular dispersions consisting of ionic lipids (liposomes) and/or of nonionic lipids.

7. Use according to any one of the preceding claims, characterized in that the composition contains, in addition, at least one dermatological ingredient chosen from humectants, preservatives, colourants, perfumes and penetrating agents.

## Patentansprüche

1. Verwendung mindestens einer Dicaffeylchinasäure als Wirkstoff für die topische Applikation, welcher der folgenden allgemeinen Formel (I) entspricht: 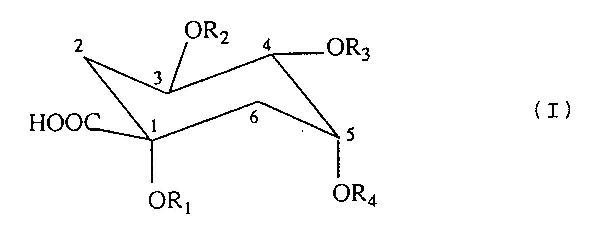 worin
R₁ R₂, R₃ und R₄ ein Wasserstoffatom oder den CAF-Rest (Kaffeesäurerest) der folgenden Formel 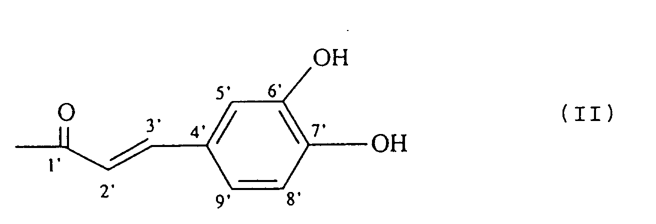 mit der Maßgabe bedeuten, daß mindestens zwei und höchstens drei Reste R₁, R₂, R₃ und R₄ den CAF-Rest, jedoch unter Ausschluß von Verbindungen, in denen R₁ und R₄ den CAF-Rest und R₂ und R₃ ein Wasserstoffatom darstellen,
oder ein Gemisch dieser Caffeylchinasäureverbindungen,
zur Herstellung einer dermatologischen Zubereitung, welche zur spezifischen Behandlung von Pigmentflecken und zum Bleichen der Haut bestimmt ist.

2. Verwendung nach Anspruch 1, dadurch gekennzeichnet, daß die Zubereitung auf topischem Weg in einem Mengenverhältnis von 0,5 bis 3 mg/cm² Hautfläche angewendet wird.

3. Verwendung nach einem der Ansprüche 1 und 2, dadurch gekennzeichnet, daß die Di- oder Tricaffeylchinasäureverbindung unter den folgenden ausgewählt wird:
- 4,5-O,O'-Dicaffeylchinasäure,
- 3,5-O,O'-Dicaffeylchinasäure,
- 1,3-O,O'-Dicaffeylchinasäure,
- 3,4-O,O'-Dicaffeylchinasäure,
- 3,4,5-O,O',O''-Tricaffeylchinasäure,
sowie deren Gemische.

4. Verwendung nach einem der vorstehenden Ansprüche, dadurch gekennzeichnet, daß die Di- bzw. Tricaffeylsäurechinasaure in einer Konzentration vorhanden ist, welche zwischen 0,01 und 15 Gew.-% in bezug auf das Gesamtgewicht der Zusammensetzung beträgt.

5. Verwendung nach einem der vorstehenden Ansprüche, dadurch gekennzeichnet, daß die Di- bzw. Tricaffeylchinasäure in der Zusammensetzung in einer Konzentration vorhanden ist, welche zwischen 0,1 und 8 Gew.-% in bezug auf das Gesamtgewicht der Zusammensetzung beträgt.

6. Verwendung nach einem der vorstehenden Ansprüche, dadurch gekennzeichnet, daß die Zubereitung in Form einer Lotio, einer Creme, einer Milch, eines Gels, einer Maske, oder in Form von vesikulären Dispersionen vorliegt, welche aus ionischen Lipiden (Liposomen) und/oder nichtionischen Lipiden bestehen.

7. Verwendung nach einem der vorstehenden Ansprüche, dadurch gekennzeichnet, daß die Zubereitung zusätzlich noch mindestens einen dermatologischen Inhaltsstoff enthält, der unter den Feuchthaltemitteln, Konservierungsmitteln, Farbstoffen, Duftstoffen und Penetrationshilfsstoffen ausgewählt ist.
